# FASCICULE DE BREVET EUROPEEN

(11) **EP 0 704 191 B1**
(45) Date de publication et mention de la délivrance du brevet: **19.12.2001**
(21) Numéro de dépôt: 95810597.5
(22) Date de dépôt: 25.09.1995
(51) Int. Cl.: A61B 17/16

(54) **Ensemble porte-fraise et fraise pour la chirurgie**
Chirurgische Anordnung mit einem Fräser und einem Halter hierfür
Surgical assembly comprising a milling cutter holder and a milling cutter

(30) Priorité: 28.09.1994 CH 293394
(43) Date de publication de la demande: 03.04.1996
(73) Titulaire: PRECIMED S.A., 2534 Orvin (CH)
(72) Inventeur: Lechot, André, CH-2534 Orvin (CH)
(74) Mandataire: Meylan, Robert Maurice

(56) Documents cités:
- FR-A- 2 281 095
- US-A- 4 621 637

## Description

L'invention concerne un ensemble porte-fraise et fraise destinés à la chirurgie dans le domaine de l'implantation de prothèses, comprenant, d'une part, un manche auquel est fixée une tête porte-fraise munie d'un dispositif de fixation de type baïonnette muni de moyens de verrouillage et, d'autre part, une fraise en forme de calotte munie de tiges radiales, distribuées régulièrement, solidaires du bord intérieur de la fraise, destinées à venir s'engager dans des crans de la baïonnette.

Un tel ensemble porte-fraise/fraise a déjà été commercialisé par le déposant. Dans cet ensemble, la fraise en forme de calotte hémisphérique est munie de trois ergots radiaux sur son bord intérieur, destinés à venir s'engager dans les crans de la baïonnette. Ceux-ci peuvent être verrouillés par des ergots placés à l'extrémité d'un coulisseau mobile axialement sur le manche du porte-fraise et ramené contre la baïonnette par l'action d'un ressort. Aux ergots du coulisseau correspondent des trous dans la baïonnette, que traversent les ergots pour fermer les crans de la baïonnette lorsque le coulisseau est plaqué contre la baïonnette. Pour ouvrir les crans de la baïonnette, il suffit d'éloigner suffisamment le coulisseau de la baïonnette. Du fait que les tiges de fixation de la fraise sont des ergots, la fraise présente une rigidité relativement faible. D'autre part, les ergots ayant une longueur limitée, une tête de porte-fraise n'est utilisable que pour des fraises ayant des diamètres bien déterminés situés dans une fourchette étroite, ce qui implique de fréquents montages et démontages de la tête du porte-fraise lors de l'utilisation de différentes fraises.

Un outil de fraisage du même type, correspondant au préambule de la revendication 1, est connu du document FR-A-2 281 095. Cet outil est destiné au façonnage de la cavité cotyloïde lors d'un remplacement par prothèse totale de l'articulation de la hanche. Il est constitué d'une fraise pouvant avoir la forme d'une hémisphère et d'une tête porte-fraise destinée à fermer la partie inférieure de la fraise de façon à ce que les particules de tissus enlevées lors de l'opération de fraisage parvenant dans la cavité intérieure de la fraise y soient retenues jusqu'à la fin de l'opération, et à être montées sur un arbre d'entraînement. Selon une forme d'exécution de cet outil, une tige unique fixée par ses deux extrémités à l'intérieur de la fraise est prévue pour la fixation de la fraise sur la tête porte-fraise. Elle coopère, par exemple, avec un système de fixation de type baïonnette dont est munie la tête porte-fraise. Cet outil de fraisage dispose d'une bonne rigidité du fait que lors de l'effort de fraisage le bord inférieur de la fraise est appuyé contre la tête porte-fraise, la tige lui conférant une stabilité supplémentaire. Cet outil présente cependant un inconvénient important qui est le risque de bourrage à l'intérieur de la fraise au cours d'une opération. Lors d'un bourrage, la fraise ne rabote plus et abîme donc l'os. Un autre désavantage présenté par cet outil est que la tête porte-fraise n'est utilisable qu'avec des fraises ayant un diamètre bien déterminé, ce qui implique, comme pour l'ensemble porte-fraise/fraise déjà commercialisé par le déposant, de fréquents démontages et montages lors de l'utilisation de différentes fraises.

Le but de la présente invention est de réaliser un ensemble fraise/porte fraise permettant de pallier les inconvénients cités précédemment et en particulier permettant l'utilisation d'une tête porte-fraise pour toutes fraises ayant un diamètre intérieur supérieur au diamètre de la tête porte-fraise.

Ce but est atteint par l'ensemble porte-fraise et fraise selon l'invention caractérisé en ce que les tiges radiales sont au moins au nombre de trois et se joignent au centre où elles sont solidaires.

La matière osseuse détachée lors de l'opération de fraisage a tendance à former une pâte et donc à rester à l'intérieur de la fraise. Munir celle-ci d'un couvercle n'est donc pas nécessaire et est même déconseillé à cause du risque de bourrage.

L'ensemble porte-fraise/fraise selon l'invention dispose du fait des tiges radiales d'une bonne résistance à la déformation permettant d'obtenir une grande précision lors de l'usinage de la cavité désirée.

Les tiges radiales allant du bord de la fraise à son centre, une tête porte-fraise peut être utilisée avec toute fraise ayant un diamètre intérieur supérieur à son diamètre. Grâce à ceci, le nombre d'outils à stériliser est diminué, ce qui permet un gain de temps. Le nombre de têtes porte-fraise étant ainsi réduit, il est plus aisé d'acquérir la certitude de leur stérilité ou non stérilité, ce qui permet de diminuer, voire d'éliminer le risque d'erreurs pouvant se révéler fatales pour le patient opéré.

Selon une variante d'exécution préférée, la baïonnette est évidée en son centre sur une certaine épaisseur pour permettre aux tiges radiales ainsi jointes de venir s'engager dans les crans de la baïonnette sans être soumis à des frottements au niveau de leurs points de raccordement. Cet évidement permet également de recueillir la matière osseuse pour les greffes. Ainsi, la présence de tiges de fixation raccordées permettant l'amélioration de la rigidité de la fraise et une plus grande précision de fraisage, on peut encore recueillir de la matière osseuse.

Le dessin annexé représente, à titre d'exemple, une forme d'exécution de l'objet de l'invention.

La figure 1 est une vue de dessous de la fraise.

La figure 2 représente le porte-fraise et la fraise vus de côté.

La figure 3 représente la partie supérieure de là tête du porte-fraise.

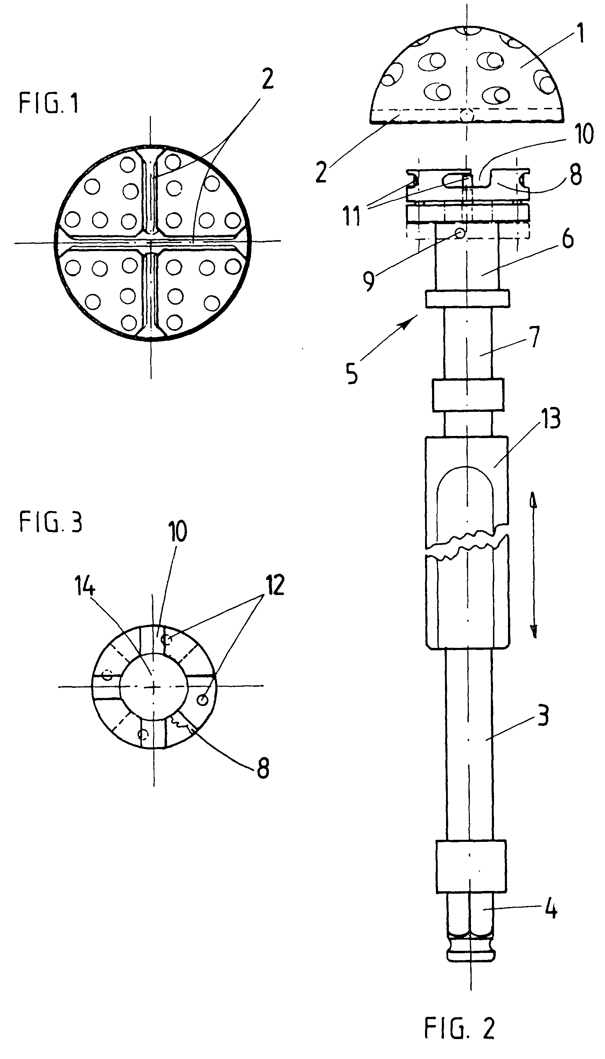

La fraise représentée aux figures 1 et 2 est constituée d'une calotte hémisphérique 1 munie d'arêtes tranchantes et de deux tiges 2 perpendiculaires l'une à l'autre, de longueur de diamètre intérieur de la calotte 1, soudées ensemble au centre de la calotte, et dont les extrémités sont soudées aux bords intérieurs de la calotte 1.

Le porte-fraise représenté à la figure 2 comprend un manche de fraise 3, un embout 4, et une tête de porte-fraises 5. L'embout 4, qui sert à fixer le manche 3 du porte-fraise sur la machine l'entraînant en rotation, est fixé à l'une des extrémités du manche 3 par une vis. La tête 5 du porte-fraise est également fixée à l'autre extrémité du manche par une vis. La tête du porte-fraise comprend un coulisseau 6 monté autour d'un axe 7 dont une extrémité est fixée au manche du porte-fraise et l'autre est pourvue d'un flasque 8 de diamètre supérieur à celui de l'axe 7. Le coulisseau 6 est poussé sur l'axe 7 par un ressort 9 qui le plaque contre le flasque supérieur 8 de l'axe. Le flasque 8 de l'axe sert de baïonnette. Il est évidé en son centre 14 sur une certaine profondeur inférieure à son épaisseur et forme ainsi une couronne. Dans cette couronne sont ménagés quatre crans de baïonnette 10 en forme de L destinés à recevoir les tiges 2 de la fraise. Le coulisseau 6 est pourvu de quatre ergots 11 parallèles à l'axe 7, auxquels correspondent quatre trous 12 dans le flasque 8, trous que traversent les ergots 11 pour fermer les crans 10 de la baïonnette et verrouiller ainsi les tiges 2 de la fraise dans la tête 5 du porte-fraise. Pour déverrouiller les tiges 2 de la fraise, il suffit d'éloigner le coulisseau 6 du flasque 8. Les tiges 2 n'étant alors plus bloquées dans les crans de la baïonnette par les ergots 11, la fraise peut être démontée de la tête porte-fraise.

Le manche 3 du porte-fraise est muni d'une poignée 13 qui coulisse le long de l'axe du manche 3 du porte-fraise.

Le nombre de tiges pourrait être différent de quatre, par exemple trois ou cinq.

Le verrouillage de la fixation à baïonnette pourrait être exécuté d'une autre manière.

Etant donné qu'une seule et même tête porte-fraise peut être utilisée pour des fraises de diamètres différents, la tête porte-fraise pourrait être fixée à demeure au manche.

## Revendications

1. Porte-fraise et fraise destinés à la chirurgie comprenant, d'une part, un manche (3) auquel est fixée une tête porte-fraise (5) munie d'un dispositif de fixation de type baïonnette (10) muni de moyens de verrouillage (6, 11) et, d'autre part, une fraise (1) en forme de calotte munie de tiges radiales (2), distribuées régulièrement, solidaires du bord intérieur de la fraise, destinées à venir s'engager dans des crans (10) de la baïonnette, **caractérisés en ce que** les tiges radiales (2) sont au moins au nombre de trois et se joignent au centre de la calotte où elles sont solidaires.

2. Porte-fraise et fraise selon la revendication 1, **caractérisés en ce que** la baïonnette a la forme d'une couronne dont le centre (14) est évidé sur une certaine épaisseur afin de pouvoir recevoir les tiges radiales (2) de la fraise en diminuant les frottements au niveau de leur point de raccordement.

3. Porte-fraise et fraise selon la revendication 2, **caractérisés en ce que** les tiges radiales de la fraise sont au nombre de quatre.

## Claims

1. Reamer spindle and reamer which are intended for surgery, comprising, on the one hand, a shank (3) to which there is fixed a reamer spindle head (5) which is equipped with a securing device of the bayonet type (10) equipped with locking means (6, 11), and, on the other hand, a cap-shaped reamer (1) which is equipped with radial rods (2) which are distributed uniformly, are integral with the inside edge of the reamer and are intended to come into engagement in catches (10) of the bayonet, **characterized in that** the radial rods (2) are at least three in number and join up at the center of the cap where they are integral.

2. Reamer spindle and reamer according to claim 1, **characterized in that** the bayonet is in the form of a collar whose center (14) is recessed to a certain thickness in order to be able to receive the radial rods (2) of the reamer, reducing the friction in the area of their connection point.

3. Reamer spindle and reamer according to claim 2, **characterized in that** the radial rods of the reamer are four in number.

## Patentansprüche

1. Fräserhalter und Fräser für die Chirurgie, mit einerseits einem Griff (3), an welchem ein Fräserhalterkopf (5) befestigt ist, der mit einer Verriegelungsmittel (6, 11) aufweisenden Befestigungvorrichtung vom Bajonett-Typ (10) versehen ist, und anderereits einem Fräser (1) in Form einer Kalotte, die mit radialen Stiften (2) versehen ist, welche regelmässig verteilt und fest am Innenrand des Fräsers angebracht und dazu bestimmt sind, in Schlitze (10) des Bajonetts einzugreifen, **dadurch gekennzeichnet, dass** die Anzahl der radialen Stifte (2) wenigstens drei beträgt und diese Stifte sich im Zentrum der Kalotte treffen, wo sie befestigt sind.

2. Fräserhalter und Fräser nach Anspruch 1, **dadurch gekennzeichnet, dass** das Bajonett die Form einer Krone hat, deren Zentrum (14) auf eine bestimmte Dicke ausgehöhlt ist, um die radialen Stifte (2) des Fräsers aufzunehmen, wobei die Reibungen in Höhe ihres Verbindungspunkts verringert werden.

3. Fräserhalter und Fräser nach Anspruch 2, **dadurch gekennzeichnet, dass** die Anzahl der radialen Stifte des Fräsers vier beträgt.
